(19) Europäisches Patentamt
European Patent Office
Office européen des brevets

(11) EP 0 867 722 A2

# (12) EUROPEAN PATENT APPLICATION

(43) Date of publication:
30.09.1998 Bulletin 1998/40

(51) Int. Cl.[6]: **G01N 33/58**, G01N 33/542, G01N 21/64

(21) Application number: 98105447.1

(22) Date of filing: 25.03.1998

(84) Designated Contracting States:
**AT BE CH DE DK ES FI FR GB GR IE IT LI LU MC NL PT SE**
Designated Extension States:
**AL LT LV MK RO SI**

(30) Priority: 27.03.1997 JP 74943/97

(71) Applicant: TAKARA SHUZO CO. LTD.
Fushimi-ku, Kyoto-shi, Kyoto-fu (JP)

(72) Inventors:
- Miyamura, Tsuyoshi
2-chome, Kusatsu-shi, Shiga-ken (JP)
- Burke, Thomas J.
Madison, Wisconsin 53719 (US)
- Bolger, Randall E.
Oregon, Wisconsin 53575 (US)
- Kato, Ikunoshin
Uji-shi, Kyoto-fu (JP)

(74) Representative:
Vossius, Volker, Dr. et al
Dr. Volker Vossius,
Patentanwaltskanzlei - Rechtsanwaltskanzlei,
Holbeinstrasse 5
81679 München (DE)

## (54) Method for measuring interaction between sugar and target

(57) An improved method for measuring an interaction between sugars and targets by a fluorescence polarization technique is provided. The improvement resides in that the sugars are involved in the form of substantially stable fluorescence-labeled sugars which are obtained by reacting specific groups (e.g., reducing groups) of the sugars with labeling fluorophores. This method can be used for the screening of sugars, particularly those derived from natural products, having an interaction with targets and the subsequent structural analysis of such sugars, or for the screening of targets having an interaction with specific sugars and the subsequent structural analysis of such targets.

Fig. 2

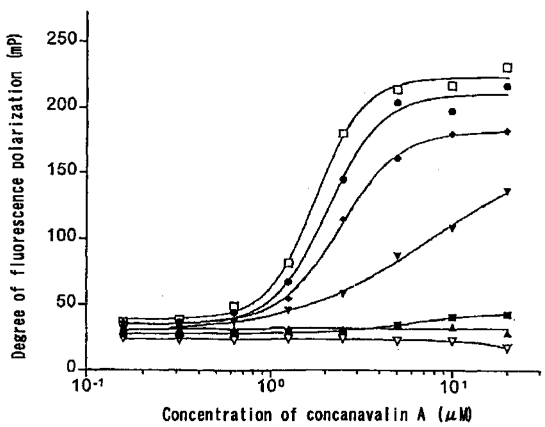




Printed by Xerox (UK) Business Services
2.16.3/3.4

## Description

### FIELD OF THE INVENTION

The present invention relates to a method for measuring an interaction between sugars and targets. More particularly, the present invention relates to a series of procedures for identifying sugars obtained from natural products, and/or targets for these sugars, in which an interaction between the sugars and the targets is measured by a fluorescence polarization technique. The present invention further relates to a kit for use in this method.

The term "interaction" as used herein refers to any action by intermolecular force arising from at least one of the electrostatic force, covalent bonding, hydrophobic bonding, van der Waal's force and hydrogen bonding, which are exerted or formed between the molecules. The force exerted by electrostatic force between the molecules may also include any force arising from electric repulsion, in addition to any force arising from electrostatic coupling. The force exerted by covalent bonding between the molecules may also include any force exerted between the dipoles when atoms having different electronegativities are covalently bonded, in addition to any force arising from coordinate bonding. The interaction may further include bonding, synthesis, degradation and any other reactions resulting from the above action.

Typical examples of the interaction may include the bonding and dissociation of ligands (e.g., signal transmitters) and receptors; those of adhesion molecules and their counterpart molecules; those of antigens and antibodies; those of enzymes and substrates, and the resulting degradation of the substrates or the resulting transfer between the substrates. Further included are the bonding and dissociation of sugars and lectins which are sugar-binding proteins; those of sugars and cells or cell fragments; those of sugars and macromolecules; and structure changes arising from non-covalent bonding, such as gelatinization of starch.

### BACKGROUND OF THE INVENTION

In recent years, it has been elucidated that sugar chains are concerned in the phenomenons such as intercellular recognition and adhesion. Many reports have also been made that sugar chains or their derivatives have physiological activity [see, e.g., Varki, A., Glycobiology, Vol. 3, 97-130 (1993)]. It has further been known that sugar chains often serve as the marker for cancer or cell differentiation [see, e.g., Varki, A., Glycobiology, Vol. 3, 97-130 (1993)], and there is no doubt that sugar chains play an important role in the cancer or cell differentiation.

To the expression of these physiological functions of sugars is essential, in many cases, an interaction between the sugar (i.e., sugar ligands) and the sugar receptors which are macromolecule receptors or binders. There is no doubt that the searching and function analysis of sugar receptors are important steps of elucidating a mechanism of the action of sugar chains and thereby developing a drug capable of stimulating or antagonize the sugar receptors.

From the viewpoint of receptors, the searching of true ligands which actually function in vivo and the screening of stronger ligands are the most effective strategies of the study. The measurement of an interaction between sugars and targets such as sugar receptors is essential to the research of sugar functions as described above.

In general, radioreceptor assays are usually used for the measurement of an interaction between ligands and receptors. The radioreceptor assays can also be used for the measurement of an interaction between sugars and targets. In the case of sugars, however, no non-specific and effective radiolabeling technique such as iodine labeling on proteins is available, and labeling by chemical synthesis is quite difficult because of their complicated structure. Thus, only a few reports are found on the actual use of a radioreceptor assay in the measurement of an interaction between sugars and sugar receptors [see, e.g., Shibuya, N. et al., FEBS Lett., Vol. 329, 75-78 (1993)].

In addition to the radioreceptor assays, fluorescence polarization techniques have been known for a long time as a method for measuring an interaction between ligands and receptors [see, e.g., Perrin, F., J. Phys. Rad., Vol. 1, 390-401 (1926)]. When this method is employed for measuring an interaction between sugars and targets, it needs fluorescent labeling of the sugars. In the previous reports, however, only synthetic products of simple oligosaccharides including up to tetrasaccharides at most have been used [see, e.g., Decastel, M. et al., Arch. Biochem. Biophys., Vol. 232, 640-653 (1984)].

Furthermore, there has recently been reported a method for measuring an interaction between sugar chains and lectins by surface plasmon resonance [see, e.g., Hutchinson, A., Anal. Biochem., Vol. 220, 303-307 (1994)]. However, this method has not yet been widely used because it requires expensive special apparatus and immobilization of either sugar chains or receptors.

Such methods that have been used so far are very convenient for the screening or purification of targets such as sugar receptors for specific purified sugars which have already been known to have some activity because the purified specific sugars should be labeled or immobilized.

However, like the case of selectin, in vivo functioning ligands have not yet been identified, although there can be found various views [see, e.g., Lasky, L., Ann. Rev. Biochem., Vol. 64, 113-139 (1995)], whereas very detailed studies

have been made on the targets. This is because the targets are proteins and the studies of proteins are remarkably promoted by the introduction of genetic engineering techniques as compared with those of sugars. In such a case, there occurs the need of screening the sugars having an interaction with the specific targets from "the living cells".

When plural molecules are taken as candidates, intermolecular interactions via sugars are not always highly specific, dissimilarly to antigen-antibody interaction. In such a case, there occurs the need of evaluating the degree of interaction by the determination of bonding constants and then making a comparison between the molecules.

In the prior art technology, however, the sugar candidates must be labeled or immobilized, which requires much effort. The measurement cannot be carried out in the homogeneous system because of immobilization and it must be made under the different conditions from the environment in nature. In the case of unknown sugars, it further needs the separation and purification of the sugars to give pure products. From this point of view, it is quite difficult to measure an interaction between sugars and targets by the conventional method.

Thus, there has hitherto been a great demand for the development of a method for selecting or purifying sugars having an interaction with targets from "the living cells".

The necessary conditions needed in any method for screening sugars having an interaction with the specific targets from natural products and then conducting the structural analysis of these sugars are as follows:

1. The sugars are labeled to serve as an index in the purification.
2. The label is stable during the purification.
3. The measurement of an interaction between the sugars and the targets can be conducted in a simple manner.
4. The label is also utilized in the measurement of an interaction with the targets.
5. The structural analysis of the purified sugars can be conducted with high sensitivity in a simple manner.

Among these conditions, fluorescence polarization techniques meet the third condition.

The principles of fluorescence polarization techniques are briefly explained below. That is, the fluorescence polarization techniques utilize the phenomenon that fluorescent molecules excited by plane-polarized light emit fluorescent light on the same plane of polarization when they keep the stationary state but the emitted fluorescent light has a different plane of polarization from that of the excitation light when the excited molecules make a motion such as rotation. The molecular motion is affected by the size of molecules. When the fluorescent molecules are large in size, they make almost no molecular motion in the excited state and the emitted light is kept being polarized. In contrast, when the fluorescent molecules are small in size, the emitted light is depolarized because of their high speed motion. Thus, the intensity of fluorescent light emitted from the fluorescent molecules excited by plane-polarized light is measured both on the original plane and on the plane perpendicular thereto, and the ratio of fluorescence intensity on both planes makes it possible to obtain some information on the motility of these molecules and the states of their being.

The degree of fluorescence polarization (P) is expressed by the following equation:

$$P = (Int_{\parallel} - Int_{\perp}) / (Int_{\parallel} + Int_{\perp})$$

wherein $Int_{\parallel}$ is the intensity of the emitted light (fluorescence) parallel to the plane of polarization of excitation light and $Int_{\perp}$ is the intensity of the emitted light perpendicular to the plane of polarization of excitation light.

The degree of fluorescence polarization is usually obtained by placing polarizing elements both on the side of excitation light and on the side of emitted light, rotating the polarizing element on the side of emitted light, and measuring two kinds of emitted light each having a polarization plane either parallel or perpendicular to the polarization plane of the excitation light. Thus, one measurement can be completed for a short time within one minute. Such a short time of measurement makes it possible to conduct the kinetic analysis of an interaction between two molecules. Furthermore, this method requires no immobilization and allows measurement in a homogeneous system.

## SUMMARY OF THE INVENTION

Under these circumstances, the present inventors have extensively studied to find a labeling method and a fluorochrome for use in labeling, which meet the remaining conditions described above, thereby completing the present invention.

That is, in the first aspect, the present invention provides an improved method for measuring an interaction between a sugar and a target by a fluorescence polarization technique, the improvement wherein the sugar is involved in the form of a fluorescence-labeled sugar which is obtained by reacting a specific group of the sugar with a labeling fluorophore.

In the second aspect, the present invention provides a kit for use in a method for measuring an interaction between a sugar and a target as described above, comprising a labeling fluorophore capable of reacting with a specific group of the sugar or comprising a fluorescence-labeled sugar which is obtained by reacting a specific group of the sugar with

a labeling fluorophore.

In the third aspect, the present invention provides a method for measuring an interaction between a sugar and a target, comprising the steps of:

(a) preparing a fluorescence-labeled sugar by labeling a specific group of the sugar with a labeling fluorophore; and
(b) measuring an interaction between the fluorescence-labeled sugar and the target by a fluorescence polarization technique.

In the fourth aspect, the present invention provides a method for searching a sugar having an interaction with a target, comprising at least one repetition of the steps:

(a) preparing a fluorescence-labeled sugar by labeling a specific group of the sugar with a labeling fluorophore;
(b) measuring an interaction between the fluorescence-labeled sugar and the target by a fluorescence polarization technique; and
(c) subjecting the fluorescence-labeled sugar to structural analysis.

In the fifth aspect, the present invention provides a method for searching a target having an interaction with a sugar, comprising at least one repetition of the steps:

(a) preparing a fluorescence-labeled sugar by labeling a specific group of the sugar with a labeling fluorophore;
(b) measuring an interaction between the fluorescence-labeled sugar and the target by a fluorescence polarization technique; and
(c) subjecting the target to structural analysis.

The measurement methods of the present invention are characterized in that a specific group of the sugar is labeled with a fluorescent agent prior to the measurement of an interaction between the sugar and the target, or a fluorescence-labeled sugar is used in the measurement. These measurement methods are further characterized in that an interaction between the sugar and the target is measured by determining the degree of fluorescence polarization of an introduced fluorochrome and the fluorescence is taken as an index in the structural analysis of the sugar.

## BRIEF DESCRIPTION OF THE DRAWINGS

Figure 1 is a chart of high performance liquid chromatography showing the separation of a mixture of fluorescence-labeled sugars obtained from kidney beans as the raw material.

Figure 2 is a graph showing a relationship between the concentration of concanavalin A and the degree of fluorescent polarization for some aliquots of fluorescence-labeled sugar fractions.

## DETAILED DESCRIPTION OF THE INVENTION

The fluorescence-labeled sugar used in the measurement methods of the present invention is not particularly limited, so long as it has been labeled with a labeling fluorophore capable of labeling a specific group of the sugar. When a fluorescence-labeled sugar is separated and purified by taking fluorescence as an index, it is preferred that the fluorescence intensity per molecule of the sugar is almost constant and only a specific group of the sugar has been labeled. On the other hand, when a fluorescence-labeled sugar is used in the measurement of an interaction between the sugars and the targets, it is preferred that fluorescent labeling has only a small influence on the stereostructure of the sugar molecules.

For example, when a labeling fluorophore capable of reacting to a hydroxy group is used, the non-specific reaction of this labeling fluorophore results in the formation of many kinds of fluorescence-labeled sugars, e.g., thirty-two kinds of fluorescence-labeled glucose in theory because even glucose has five hydroxy groups. Thus, the use of a fluorescence labeling method in which plural derivatives are obtained from one sugar causes a trouble, for example, in the structural analysis of the sugar. The non-specific labeling may cause the introduction of fluorochromes into the crucial sites relating to an interaction between the sugars and the targets, which will affect the analysis of such an interaction in some cases. When the methods of the present invention is directed to sugars present in natural products, there is a possibility that components other than the sugars, such as proteins or nucleic acids, may be labeled.

In the case of monosaccharides or oligosaccharides with simple structure, the selective labeling of only a specific group of the sugar molecules with a fluorochrome can also be achieved by a technique of the organic synthesis; however, in the case of sugar molecules with unknown structure present in natural products, the selective labeling of only a specific group is quite difficult.

It is, therefore, preferred that the step of "reacting a specific group of the sugar with a labeling fluorophore" in the present invention requires no additional step such as protecting the other groups of the sugar.

The fluorescence-labeling of a sugar including the step of protecting the other groups of the sugar may be achieved, for example, by protecting the groups, into which fluorochromes are not to be introduced, and then introducing fluorochromes into the desired groups, followed by the removal of the protecting groups; or by protecting the desired groups, into which fluorochromes are to be introduced, and then inactivating the other groups, followed by the introduction of fluorochromes into the desired groups.

The fluorescence-labeling of a sugar without protecting any group of the sugar is not particularly limited, and sugar-specific labeling is suitable because of no reaction with the other components in the living cells, such as proteins. For example, there can be mentioned labeling of the reducing end of a sugar.

Examples of such reducing end may include, for example, carbonyl groups and free aldehyde groups present in the sugar chain terminals. The labeling reaction at the sugar chain terminals is particularly preferred in the present invention because of almost no influence on the stereostructure of the sugar. The reaction with the reducing groups of a sugar can be achieved, for example, by reductive amination or hydrazide formation.

The sugar to be labeled by reductive amination is not particularly limited, so long as it has a reducing group terminal or an aldehyde group capable of specifically reacting with an amino group. Two or more amino groups per molecule of the sugar may be introduced in the reaction. The sugar may be separated from the living cells or artificially prepared by chemical synthesis. The sugar to be fluorescence-labeled may be homogeneous or in the form of a mixture, or it may contain any coexisting substance other than the sugar, such as proteins, salts or lipids. The reaction conditions such as solvents, counter ions, temperatures and time for the labeling by reductive amination are not particularly limited, so long as the condensation of a sugar with a fluorescent substance to form a Schiff base can be effected concurrently with or separately from the reduction of the Schiff base to form a stable fluorescent sugar chain. Preferred is any reaction causing no elimination of sialic acid and giving high reaction yield.

The labeling fluorophore to be used is not particularly limited, so long as it has at least one amino group.

In the case where the sugars contained in natural products are separated and purified, and then subjected to the measurement of an interaction with targets and further to the structural analysis of the sugar molecules, it requires a long time because the sugars have complicated structure. It is, therefore, preferred that the fluorescence-labeled sugar is stable. The term "substantially stable fluorescence-labeled sugar" as used herein refers to those having a stable fluorescence intensity during the above series of procedures. The term "substantially stable" as used herein refers to, for example, the stability to visible light, in addition to the stability to decomposition in an aqueous solution. In general, the experimental procedures are performed in the inside of a laboratory, so that the labeled sugar is exposed to light. Therefore, the "substantially stable" fluorescence-labeled sugar may include, for example, those having a pyridyl group as the fluorochrome. The pyridyl group has about 100% fluorescence intensity as the start of irradiation, even if it is irradiated with 4000 lux light of a fluorescent lamp for 16 hours. In contrast, the fluorescein group widely used as the fluorochrome has reduced fluorescence intensity to about 20%, when allowed to stand under the same conditions. When the fluorescein group is used as the labeling fluorochrome, necessary steps should be taken for shading during the procedures. This is, however, inconsistent with the purification of a substance with fluorescence as an index because the irradiation of light is required in the measurement of fluorescent light emitted from the substance.

Therefore, the term "substantially stable" fluorescence-labeled sugar as used herein refers to sugars labeled with a fluorochrome having 20% or more fluorescence intensity, even if irradiated with 4000 lux light of a fluorescent lamp for 16 hours.

The fluorescence-labeled sugar meeting the above conditions may be a sugar having a fluorochrome which is derived from at least one compound selected from the group consisting of pyridine, benzene, coumarin, naphthalene, anthracene and pyrene. Such a fluorescence-labeled sugar may be obtained, for example, by reacting at least one selected from the group consisting of 2-aminopyridine, 2-aminobenzamide, 7-amino-4-methylcoumarin, 8-amino-naphthalene-1,3,6-trisulfonic acid, 5-dimethylaminonaphthalene-1-(N-(2-aminomethyl))sulfonamide and 1-aminopyrene-3,6,8-trisulfonic acid to a sugar.

The sugar used in the present invention may be any one selected from the group consisting of monosaccharides, oligosaccharides, polysaccharides, and mixtures thereof. Furthermore, it may be derived from glycolipids, glycopeptides or nucleic acids.

The target having an interaction with a sugar may be any one selected from the group consisting of proteins, lipids, carbohydrates, nucleic acids, and conjugates thereof. For example, it may be cells or cell fragments. Alternatively, it may be artificial substances or artificial combinations of plural substances. Furthermore, it may be homogeneous or in the form of a mixture.

In the methods of the present invention, it is preferred that substances other than the labeled sugars, such as remaining reagents, are removed after the fluorescent labeling. It is further preferred that separation is carried out by a technique such as chromatography or electrophoresis.

The apparatus for measuring the degree of fluorescence polarization is not particularly limited, so long as it is provided with a source of excitation light for exciting a fluorochrome, polarizing elements for obtaining excitation polarized light and fluorescent polarized light, a detector for converting the amount of fluorescence into the amount of electricity, such as a photoelectric tube or a photomultiplier tube, and a meter or a recorder for reading the amount of electricity converted by the detector. The conditions for fluorescence measurement are not particularly limited.

In the second aspect, the present invention provides a kit for use in a method for measuring an interaction between a sugar and a target by a fluorescence polarization technique.

The kit comprises, as essential components, a labeling fluorophore, such as 2-aminopyridine, to form a fluorescence-labeled sugar as described above. More particularly, for example, when the sugar is obtained from natural products, the kit may comprise, as additional components, a reagent for separating the sugar from proteins and a carrier for use in chromatography to isolate the sugar. In another example, the kit may comprise, as an essential component, a fluorescence-labeled sugar as described above.

These components can be prepared in liquid or solid form by any known method.

In a preferred example, the above method of the present invention can be used to measure the degree of interaction between a sugar and a target. More particularly, such a method may comprise the steps of:

(a) preparing a substantially stable fluorescence-labeled sugar by labeling a sugar with a fluorophore; and
(b) measuring an interaction between the isolated fluorescence-labeled sugar and the target by a fluorescence polarization technique.

The step (a) is conducted for the preparation of a fluorescence-labeled sugar. The sugar to be used in this step is not particularly limited, and it may be a synthetic sugar or a sugar present in natural products. The sugar derived from natural products can be fluorescence-labeled after separated and purified from natural products. The fluorescence-labeled sugar to be prepared in this step should be substantially stable. The fluorescence-labeled sugar may also be prepared by fluorescence labeling of a mixture of sugars present in natural products and then purifying the labeled sugars with an index of fluorescence by a technique such as column chromatography.

The step (b) is conducted for the measurement of an interaction between the fluorescence-labeled sugar obtained in step (a) and a target by a fluorescence polarization technique. The measuring apparatus is not particularly limited, so long as it is suitable for the measurement of fluorescence polarization of fluorescence-labeled sugars.

The interaction between the sugar molecules and the target molecules to be measured is not always highly specific as compared with antigen-antibody reaction. To find the most suitable combination, the degree of interaction should be evaluated. As an index to show the degree of interaction, for example, $EC_{50}$ can be used, which is a half value of the minimum target concentration giving the maximum degree of fluorescence polarization.

In another preferred example, the above method of the present invention can also be used for the screening of sugar molecules to be measured for their interaction with a target. More particularly, such a method may comprise the steps of:

(a) preparing a substantially stable fluorescence-labeled sugar by labeling a sugar with a fluorophore;
(b) measuring an interaction between the fluorescence-labeled sugar and the target by a fluorescence polarization technique; and
(c) subjecting the fluorescence-labeled sugar to structural analysis.

The step (a) is conducted for the preparation of a fluorescence-labeled sugar. The sugar to be used in this step is not particularly limited, and it may be a synthetic sugar or a sugar present in natural products.

The step (b) is conducted for the measurement of an interaction between the fluorescence-labeled sugar obtained in step (a) and a target by a fluorescence polarization technique. The measuring apparatus is not particularly limited, so long as it is suitable for the measurement of fluorescence polarization of fluorescence-labeled sugars. In this step, the measurement of fluorescence polarization is to be conducted for many samples, and it is, therefore, preferred that one measurement is completed for a short time. From this point of view, good results can be obtained by the replacement of a light source, polarizing elements and other parts in a measuring apparatus (e.g., BEACON™ system, PanVera Co.) ensuring that one measurement is completed for a short time within one minute, according to the measurement conditions for the fluorescence-labeled sugar used.

The step (c) is conducted for the structural analysis of sugar molecules having an interaction with the target. When the sugar is obtained from natural products, this step may comprise separating and purifying the sugar by a technique such as chromatography.

In general, the purification of unlabeled sugars, particularly sugar chains, is difficult from a viewpoint of detection (e.g., lack of any appropriate chromophore) or in that a means of purification is limited because ordinary natural sugar chains have no electric charge. It is, therefore, preferred to employ a labeling method allowing to select purification method from a wider range of choice. Furthermore, it is preferred to use fluorophores which are stable under the reaction conditions used in the study of sugar chain structure, such as methylation, acid or alkali hydrolysis, periodate oxidation and hydrazinolysis, and which can give, for example, an appropriate electric charge or hydrophobicity to the sugar. From this point of view, pyridyl group-containing fluorochromes meet the above conditions and are particularly suitable in this step.

The identification of separated and purified fluorescence-labeled sugars can be achieved by structural analysis with a physical or chemical technique such as methylation analysis, nuclear magnetic resonance or mass spectrometry, or by comparison of physical properties with the existing standard product of fluorescence-labeled sugars. Therefore, the use of a pyridylamino-labeled sugar is preferred in this step because the pyridylamino-labeled sugar is relatively stable under the reaction conditions used in the study of sugar chain structure, the range of choice in determining a means of purification can be extended by providing a sugar chain with an electric charge and hydrophobicity to an appropriate extent through fluorescence-labeling, and a number of the standard products are available.

In still another preferred example, the above method of the present invention can also be used to search an unknown target having an interaction with a sugar. More particularly, such a method may comprise at least one repetition of the steps:

(a) preparing a substantially stable fluorescence-labeled sugar by fluorescent labeling the sugar with a fluorophore;
(b) measuring an interaction between the fluorescence-labeled sugar and the target by a fluorescence polarization technique; and
(c) subjecting the target to structural analysis.

The step (a) is conducted for the preparation of fluorescence-labeled sugars. The sugar sample used in the present invention is not particularly limited, and it may be a synthetic sugar or a sugar present in natural products. The sugar derived from natural products can be fluorescence-labeled after separated and purified from natural products. The fluorescence-labeled sugar to be prepared in this step should be substantially stable. The fluorescence-labeled sugar can also be prepared by fluorescence-labeling of a sugar present in natural products and then purifying the labeled sugar with an index of fluorescence by means of a technique such as column chromatography.

The step (b) is conducted for the measurement of an interaction between the fluorescence-labeled sugar obtained in step (a) and the target by a fluorescence polarization technique. The measuring apparatus is not particularly limited, so long as it is suitable for the measurement of fluorescence polarization of fluorescence-labeled sugars. In this step, the measurement of fluorescence polarization is to be conducted for many samples, and from this point of view, an improved apparatus of the BEACON™ system as described above can be preferably used.

The step (c) is conducted for the identification of a target. When the target is obtained from natural products, this step may comprise separation and purification involved therein. The separation and purification can be achieved by ordinary procedures such as centrifugation, precipitation and chromatography. The structural analysis can be achieved, for example, by the sequence determination method for amino acid utilizing the Edman degradation [Edman, P., Arch. Biochem. Biophys., Vol. 22, 475 (1949)] when the target is a protein, or by the dideoxy terminal chain reaction method [Sanger, F., Nicklen, S., and Coluson, A.R., Proc. Natl. Acad. Sci. USA. Vol. 74, 5463 (1977)] when the target is DNA.

The present invention will be further illustrated by the following examples; however, the present invention is not limited to these examples.

Example 1

Stability of labeling fluorophores

When labeling fluorophores for reductive amination are used, the excitation and emission wavelengths and the emission intensity are not drastically changed after the reaction with a sugar. For the measurement of photostability, therefore, such labeling fluorophores are used. The following seven labeling fluorophores were selected.

1. 2-Aminopyridine (AP) (Nacalai Tesque Inc.)
2. 7-Amino-4-methylcoumarin (AC) (Sigma Chemical Company)
3. 8-Amino-2-naphthol (AN) (Aldrich Chemical Company, Inc.)
4. 5-((5-aminopentyl)thioureidyl)fluorescein (AF) (Molecular Probes Inc.)
5. 5-Dimethylaminonapnthalene-1-(N-(2-minoethyl))sulfone (AD) (Molecular Probes Inc.)
6. 2-Aminobenzamide (AB) (Oxford GlycoSystems Ltd.)
7. 8-Amino-naphthalene-1,3,6-trisulfonic acid (ANTS) (Molecular Probes Inc.)

Each of the above reagents was dissolved in 10 mM Tris-HCl buffer, pH 7.5, to a concentration of 1 μM, and 1 ml of each solution was placed in a test tube of hard glass (size: diameter, 12 mm and length, 120mm) and allowed to

stand at 25°C under a white fluorescent lamp. At this time, the illumination intensity in the vicinity of each solution was measured by an illumination photometer to be about 4000 lux. For controls, test tubes containing the same solutions were prepared, but they were shaded with aluminum foil and stored under the same conditions as described above. After 16 hours, the fluorescence of each solution was measured by fluorescent spectrophotometer RF-540 (Shimazu Corp.). The excitation and emission wavelengths used in the measurement of fluorescence of the solutions of each fluorescent substance, and residual fluorescence (i.e., ratio of the relative degree of fluorescence for the exposed solution to the relative degree of fluorescence for the shaded solution) are shown in Table 1.

Table 1

| Labeling fluorophore | Excitation wavelength (nm) | Emission wavelength (nm) | Residual fluorescence (%) |
|---|---|---|---|
| AP | 290 | 355 | 98 |
| AC | 340 | 445 | 68 |
| AN | 300 | 445 | 84 |
| AF | 495 | 530 | 17 |
| AD | 330 | 530 | 97 |
| AB | 315 | 420 | 96 |
| ANTS | 355 | 510 | 96 |

Example 2

Detection of sugars having an interaction with concanavalin A contained in kidney beans

1. Labeling of sugars contained in kidney beans with fluorophore

First, 1 mg of glycopeptides obtained by the thorough digestion of de-fatted kidney bean powder with actinase E (Kaken Pharmaceutical Co., Ltd.) was subjected to hydrazinolyis according to the method of Takasaki et al. [Takasaki, S. et al., Meth. in Enzymol., Vol. 83, 263-268 (1982)] to give a mixture of free sugars. The resulting mixture was freeze-dried, and then labeled with 2-aminopyridine (Nacalai Tesque Inc.) according to the method of Kondo et al. [Kondo, A. et al., Agric. Biol. Chem., Vol. 54, 2169-2170 (1990)]. The remaining reagents were removed by size exclusion chromatography on Sephadex G-15 (Pharmacia, AB) to give a mixture of fluorescence- labeled sugars. The mixture of fluorescence-labeled sugars was concentrated to dryness, and the residue was dissolved in 40 μl of distilled water. Then, 20 μl of the solution was subjected to high performance liquid chromatography under the following conditions, and seven fractions A to G were collected. The results of separation are shown in Figure 1.

In Figure 1, the ordinate represents the relative fluorescence intensity and the abscissa represents the retention time (min). The yield of each fraction was as follows: A, 1.6 nmol; B, 2.6 nmol; C, 2.2 nmol; D, 2.9 nmol; E, 6.9 nmol; F, 4.6 nmol; and G, 6.9 nmol.

Conditions:

Column: Asahi pak $NH_2$P-50 (Showa Denko K.K.),
Eluent A: a mixture containing 75 parts by volume, acetonitrile; 15 parts by volume, distilled water; and 10 parts by volume, 500 mM acetic acid-triethylamine (pH 7.3),
Eluent B: a mixture containing 50 parts by volume, acetonitrile; 40 parts by volume, distilled water; and 10 parts by volume, 500 mM acetic acid-triethylamine (pH 7.3),
Flow rate: 1 ml/min.
Column temperature: 40°C
Elution: a sample is introduced into the column equilibrated with eluent A, and the content of eluent B is increased proportionally with time from 0% to 50% in 50 min.
Detection: Fluorescence detection (excitation wavelength, 310 nm; and emission wavelength, 380 nm)

Apparatus:

Pump: LC-6A (Shimazu Corp.)
Fluorescence detector: RF-535 (Shimazu Corp.)
Column oven: CTO-6A (Shimazu Corp.)

2. Fabrication of an apparatus for measuring the degree of fluorescence polarization

BEACON™ 2000 (PanVera Corp.) was modified so as to be adapted for the detection of pyridylamino-labeled substances. That is, the light source was replaced with a mercury-vapor lamp capable of emitting light having a wavelength in the ultraviolet region, and the polarizing elements were changed to those of the Glan-Taylor type.

3. Measurement of an interaction between purified fluorescence-labeled sugars and concanavalin A

Seven fractions of fluorescence-labeled sugars prepared in the above item 1 were freeze-dried to remove water, acetonitrile and eluents-derived salts, and then used for the subsequent measurement of an interaction. The measurement of the degree of fluorescence polarization was conducted as follows.

First, concanavalin A (Honen Oil Co., Ltd.) was dissolved in the binding buffer (100 mM Tris-HCl buffer, pH 7.9, containing 1 mM calcium chloride and 1 mM manganese chloride) to a final concentration of 20 μM. This solution was 2-fold diluted with the binding buffer, and the dilution thus obtained was further 2-fold diluted with the binding buffer. This operation was repeated 7 times to prepare serial dilutions of concanavalin A at 8 levels ranging from 20 μM to 156 μM.

In each of the eight solutions of concanavalin A in various concentrations, each fluorescence-labeled sugar fraction was dissolved to a concentration of 1 μM. After the dissolution, the solutions were allowed to stand at room temperature for 30 minutes, and the reaction mixtures thus obtained were used each in a volume of 100 μl to measure the degree of fluorescence polarization with a modified apparatus of the BEACON™ system as described in the above item 2, which was equipped with filters for excitation at 310 nm and emission at 390 nm.

Figure 2 shows a change in the degree of fluorescence polarization by an interaction between each fluorescence-labeled sugar fraction and concanavalin A. The ordinate represents the degree of fluorescence polarization (mP) and the abscissa represents the concentration of concanavalin A in each reaction mixture (μM). In this figure, solid squares indicate the results obtained from fluorescence-labeled sugar fraction A; open inverted triangles, fluorescence-labeled sugar fraction B; closed triangles, fluorescence-labeled sugar fraction C; closed inverted triangles, fluorescence-labeled sugar fraction D; closed rhombi, fluorescence-labeled sugar fraction E; closed circles, fluorescence-labeled sugar fraction F; and open squares, fluorescence-labeled sugar fraction G. The binding constant concanavalin A in each of the seven fluorescence-labeled sugar fractions A to G at the concentration of 1 μM is shown in Table 2. In particular, fractions A, B and C exhibited no maximum in the degree of fluorescence polarization over the range of concentration of concanavalin A used in the measurement; it was, therefore, impossible to calculate $EC_{50}$.

TABLE 2

| Fraction | A | B | C | D | E | F | G |
|---|---|---|---|---|---|---|---|
| $ED_{50}$(μM) | -* | -* | -* | 7.733 | 2.410 | 2.058 | 1.762 |

*: - means the impossibility to calculate $EC_{50}$.

As can be seen from Table 2, among the four fractions allowing to calculate $EC_{50}$, fraction G exhibited the lowest value. This makes it clear that among the seven fluorescence-labeled sugar fractions derived from kidney beans, fraction G is most liable to make a binding to concanavalin A.

As a control experiment, the measurement was conducted in the same manner as described above, except that only a labeling fluorophore, i.e., 2-aminopyridine, was used in place of the fluorescence-labeled sugar fractions. Similarly to the above cases of fluorescence-labeled sugar fractions A, B and C, no maximum was observed in the degree of fluorescence polarization, even if the concentration of concanavalin A is increased.

4. Structural analysis of fluorescence-labeled sugars

By two dimensional mapping using high performance liquid chromatography [Tomiya, N. et al., Anal. Biochem., Vol. 171, 73-90 (1988)], the sugar faction G, which was revealed by the measurement by a fluorescence polarization technique to have the property of binding to concanavalin A, has a structure of the formula:

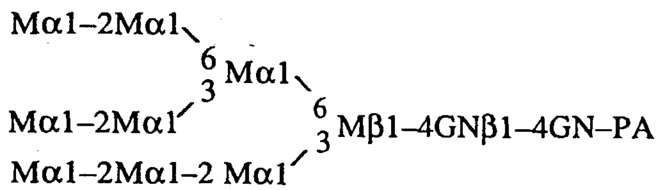

wherein M is mannose, GN is N-acetylglycosamine, GN-PA is pyridylaminated N-acetylglycosamine, α1-2, α1-3, α1-6 and β1-4 are the modes of binding between monosaccharides.

The pyridylaminated N-acetylglycosamine has a structure of the formula:

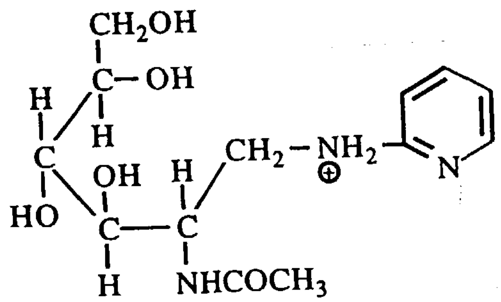

As described above, the detection of a sugar chain having the property of binding to concanavalin A from the mixture of kidney bean-derived sugar chains was successfully achieved by a fluorescence polarization technique and the structure of the sugar chain was easily determined.

According to the present invention, it becomes possible to conduct the screening of sugars, particularly derived from natural products, having an interaction with a target and the structural analysis of such sugars in a simple manner. In the opposite way, the screening of targets having an interaction with a particular sugar can also be conducted without additional procedure such as immobilization of the sugar.

## Claims

1. An improved method for measuring an interaction between a sugar and a target by a fluorescence polarization technique, the improvement wherein the sugar is involved in the form of a fluorescence-labeled sugar which is obtained by reacting a specific group of the sugar with a fluorophore.

2. A method according to claim 1, wherein the reaction of the specific group of the sugar with the labeling fluorophore is effected without protecting the specific group of the sugar.

3. A method according to claim 1, wherein the specific group of the sugar is a reducing group of the sugar.

4. A method according to claim 1, wherein the labeling fluorophore has at least one amino group.

5. A method according to claim 1, wherein the fluorescence-labeled sugar is substantially stable.

6. A method according to claim 5, wherein the fluorescence-labeled sugar is substantially stable to visible light.

7. A method according to claim 6, wherein the fluorescence-labeled sugar is substantially more stable to visible light than that having fluorescein as a fluorochrome.

8. A method according to claim 7, wherein the fluorescence-labeled sugar has a fluorochrome which is derived from

at least one compound selected from the group consisting of pyridine, benzene, coumarin, naphthalene, anthracene and pyrene.

9. A method according to claim 8, wherein the fluorescence-labeled sugar is obtained by reacting a sugar with a labeling fluorophore selected from the group consisting of 2-aminopyridine, 2-aminobenzamide, 7-amino-4-methylcoumarin, 8-amino-naphthalene-1,3,6-trisulfonic acid, 5-dimethylaminonaphthalene-1-(N-(2-aminomethyl))sulfonamide and 1-aminopyrene-3,6,8-trisulfonic acid.

10. A method according to claim 1, wherein the fluorescence- labeled sugar is obtained by fluorescence-labeling a sugar selected from the group consisting of monosaccharides, oligosaccharides, polysaccharides, and mixtures thereof.

11. A method according to claim 1, wherein the target is at least one selected from the group consisting of proteins, lipids, sugars, nucleic acids, and conjugates thereof.

12. A kit for use in a method for measuring an interaction between a sugar and a target as set forth in claim 1, comprising a labeling fluorophore capable of reacting with a specific group of the sugar.

13. A kit according to claim 12, wherein the labeling fluorophore requires no protection of a specific group of the sugar in the reaction with the specific group of the sugar.

14. A kit according to claim 12, wherein the specific group of the sugar is a reducing group of the sugar.

15. A kit according to claim 12, wherein the labeling fluorophore has at least one amino group.

16. A kit according to claim 12, wherein the labeling fluorophore gives a fluorescence-labeled sugar which is substantially stable, by reacting the labeling fluorophore with the specific group of the sugar.

17. A kit according to claim 16, wherein the labeling fluorophore gives a fluorescence-labeled sugar which is substantially stable to visible light, by reacting the labeling fluorophore with the specific group of the sugar.

18. A kit according to claim 17, wherein the labeling fluorophore gives a fluorescence-labeled sugar which is substantially more stable to visible light than that having fluorescein as a fluorochrome.

19. A kit according to claim 18, wherein the labeling fluorophore is derived from at least one compound selected from the group consisting of pyridine, benzene, coumarin, naphthalene, anthracene and pyrene.

20. A kit according to claim 19, wherein the labeling fluorophore is selected from the group consisting of 2-aminopyridine, 2-aminobenzamide, 7-amino-4-methylcoumarin, 8-aminonaphthalene-1,3,6-trisulfonic acid, 5-dimethylaminonaphthalene-1-(N-(2-aminomethyl))sulfonamide and 1-aminopyrene-3,6,8-trisulfonic acid.

21. A kit for use in a method for measuring an interaction between a sugar and a target as set forth in claim 1, comprising a fluorescence-labeled sugar which is obtained by reacting a specific group of the sugar with a labeling fluorophore.

22. A kit according to claim 21, wherein the reaction of the specific group of the sugar with the labeling fluorophore is effected without protecting the specific group of the sugar.

23. A kit according to claim 21, wherein the specific group of the sugar is a reducing group of the sugar.

24. A kit according to claim 21, wherein the labeling fluorophore has at least one amino group.

25. A kit according to claim 21, wherein the fluorescence-labeled sugar is substantially stable.

26. A kit according to claim 25, wherein the fluorescence-labeled sugar is substantially stable to visible light.

27. A kit according to claim 26, wherein the fluorescence-labeled sugar is substantially more stable to visible light than that having fluorescein as a fluorochrome.

**28.** A kit according to claim 27, wherein the fluorescence-labeled sugar has a fluorochrome which is derived from at least one compound selected from the group consisting of pyridine, benzene, coumarin, naphthalene, anthracene and pyrene.

**29.** A kit according to claim 28, wherein the fluorescence-labeled sugar is obtained by reacting a sugar with a labeling fluorophore selected from the group consisting of 2-aminopyridine, 2-aminobenzamide, 7-amino-4-methylcoumarin, 8-amino-naphthalene-1,3,6-trisulfonic acid, 5-dimethylaminonaphthalene-1-(N-(2-aminomethyl))sulfonamide and 1-aminopyrene-3,6,8-trisulfonic acid.

**30.** A kit according to claim 21, wherein the fluorescence-labeled sugar is obtained by fluorescence-labeling a sugar selected from the group consisting of monosaccharides, oligosaccharides, polysaccharides, and mixtures thereof.

**31.** A method for measuring an interaction between a sugar and a target, comprising the steps of:

(a) preparing a fluorescence-labeled sugar by labeling a specific group of the sugar with a labeling fluorophore; and
(b) measuring an interaction between the fluorescence-labeled sugar and the target by a fluorescence polarization technique.

**32.** A method for searching a sugar having an interaction with a target, comprising at least one repetition of the steps:

(a) preparing a fluorescence-labeled sugar by labeling a specific group of the sugar with a labeling fluorophore;
(b) measuring an interaction between the fluorescence-labeled sugar and the target by a fluorescence polarization technique; and
(c) subjecting the fluorescent labeled sugar to structural analysis.

**33.** A method for searching a target having an interaction with a sugar, comprising at least one repetition of the steps:

(a) preparing a fluorescence-labeled sugar by labeling a specific group of the sugar with a labeling fluorophore;
(b) measuring an interaction between the fluorescence-labeled sugar and the target by a fluorescence polarization technique; and
(c) subjecting the target to structural analysis.

Fig. 1

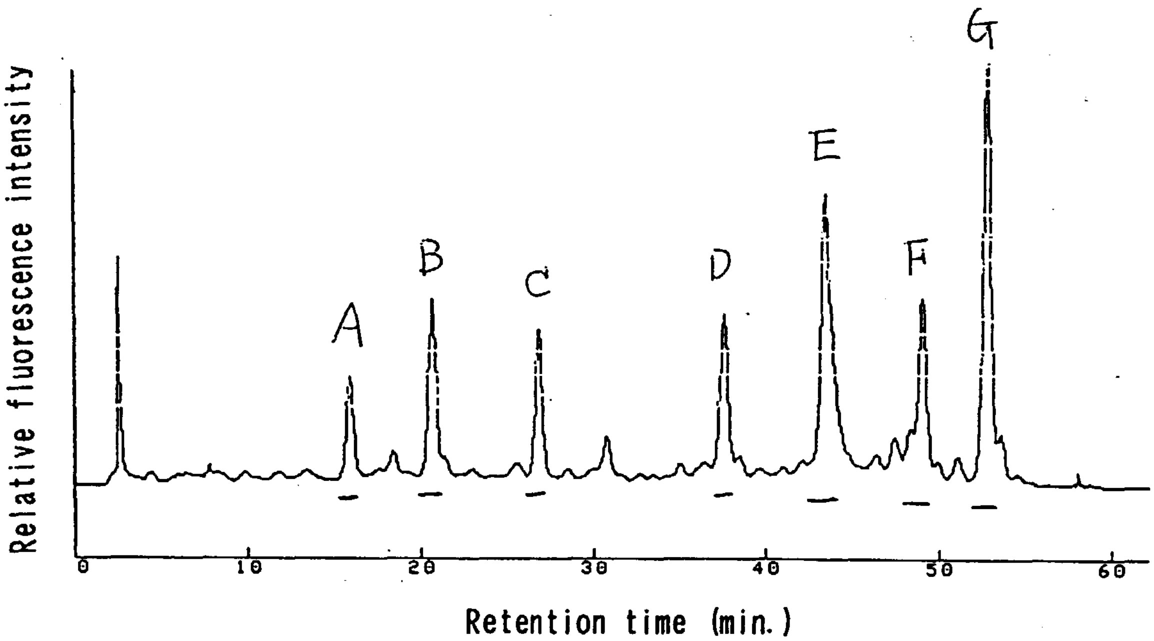
Relative fluorescence intensity
A
B
C
D
E
F
G
0
10
20
30
40
50
60
Retention time (min.)

Fig. 2

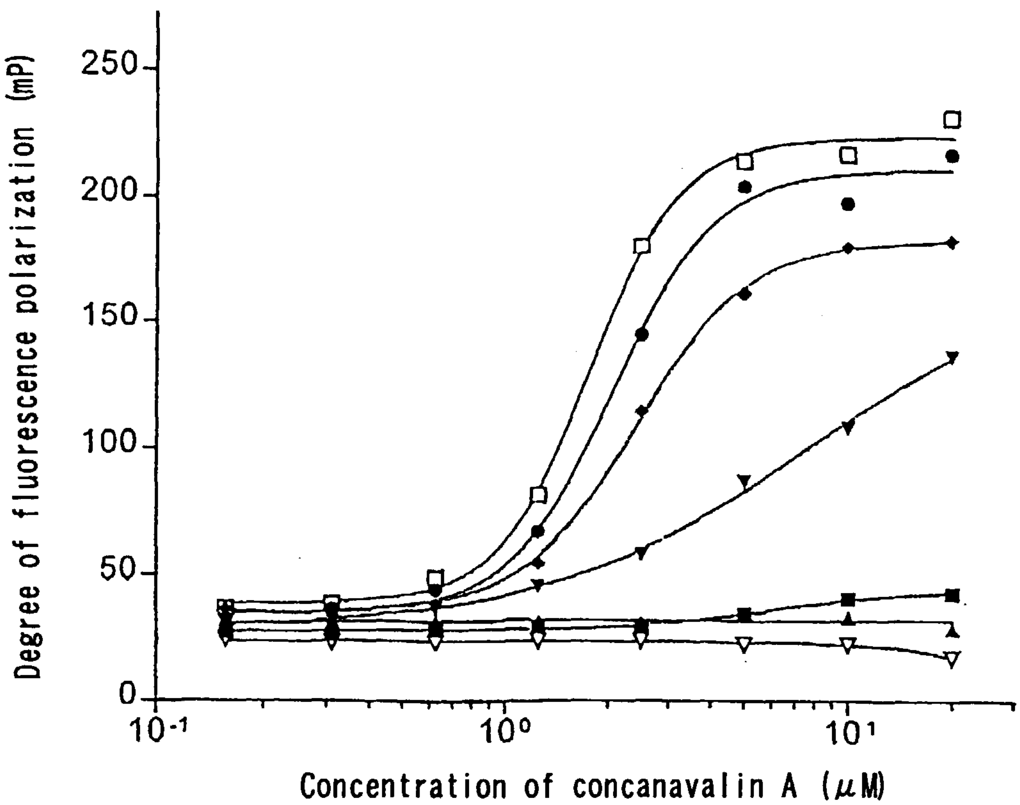
Degree of fluorescence polarization (mP)
250
200
150
100
50
0
10-1
10⁰
10¹
Concentration of concanavalin A (μM)